# EUROPEAN PATENT SPECIFICATION

(11) **EP 1 807 445 B1**
(45) Date of publication and mention of the grant of the patent: **28.01.2015**
(21) Application number: 05805329.9
(22) Date of filing: 21.10.2005
(51) Int. Cl.: C07K 14/245, C12P 13/04

(54) **METHOD FOR PRODUCING L-AMINO ACIDS USING BACTERIA OF THE ENTEROBACTERIACEAE FAMILY**
VERFAHREN ZUR HERSTELLUNG VON L-AMINOSÄUREN DURCH BAKTERIEN DER FAMILE ENTEROBAKTERIACEAE
METHODE DE PRODUCTION D'ACIDES AMINES DE TYPE L AU MOYEN DE BACTERIES DE LA FAMILLE DES ENTEROBACTERIACEES

(30) Priority: 22.10.2004 RU 2004130954
(43) Date of publication of application: 18.07.2007
(73) Proprietor: Ajinomoto Co., Inc., Tokyo 104-8315 (JP)
(72) Inventor: RYBAK, Konstantin Vyacheslavovich, Moscow 117149 (RU); SLIVINSKAYA, Ekaterina Aleksandrovna, Moscow 103055 (RU); SAVRASOVA, Ekaterina Alekseevna, Moscow 107370 (RU); AKHVERDIAN, Valeriy Zavenovich, Moscow 117593 (RU); KLYACHKO, Elena Vitalievna, Moscow 119034 (RU); MASHKO, Sergei Vladimirovich, Moscow 105043 (RU); DOROSHENKO, Vera Georgievna, Moscow 117628 (RU); AIRIKH, Larisa Gotlibovna, Moscow region 142172 (RU); LEONOVA, Tatyana Viktorovna, Moscow 123481 (RU); GUSYATINER, Mikhail Markovich, Moscow 117648 (RU); VOROSHILOVA, Elvira, Borisovna, Moscow 117648 (RU); KOZLOV, Yury Ivanovich, Moscow 117574 (RU); HARA, Yoshihiko, Kawasaki-shi, Kanagawa 210-8681 (JP); UEDA, Takuji, Kawasaki-shi, Kanagawa 210-8681 (JP)
(74) Representative: Hoffmann Eitle
(86) International application number: PCT/JP2005/019840
(87) International publication number: WO 2006/043730

(56) References cited:
- JP-A- 2004 254 694
- US-A- 5 175 107
- US-A- 5 705 371
- DAVIS E O ET AL: "THE CLONING AND DNA SEQUENCE OF THE GENE XYLE FOR XYLOSE-PROTON SYMPORT IN ESCHERICHIA COLI K12" JOURNAL OF BIOLOGICAL CHEMISTRY, AMERICAN SOCIETY OF BIOLOCHEMICAL BIOLOGISTS, BIRMINGHAM,, US, vol. 262, no. 29, 15 October 1987 (1987-10-15), pages 13928-13932, XP002924537 ISSN: 0021-9258
- DATABASE BIOSIS [Online] BIOSCIENCES INFORMATION SERVICE, PHILADELPHIA, PA, US; 2001, HEALY F G ET AL: "Development of ethanologenic Escherichia coli for the simultaneous utilization of glucose and xylose" XP002368205 Database accession no. PREV200200223084 & ABSTRACTS OF THE GENERAL MEETING OF THE AMERICAN SOCIETY FOR MICROBIOLOGY, vol. 101, 2001, page 536, 101ST GENERAL MEETING OF THE AMERICAN SOCIETY FOR MICROBIOLOGY; ORLANDO, FL, USA; MAY 20-24, 2001 ISSN: 1060-2011
- NICHOLS B S DIEN R J BOTHAST N N: "Use of catabolite repression mutants for fermentation of sugar mixture to ethanol", APPLIED MICROBIOLOGY AND BIOTECHNOLOGY, SPRINGER VERLAG, BERLIN, DE, vol. 56, no. 1-2, 1 July 2001 (2001-07-01) , pages 120-125, XP008158218, ISSN: 0175-7598, DOI: 10.1007/S002530100628

### SEQUENCE LISTING

<110> Ajinomoto Co., Inc.
<120> METHOD FOR PRODUCING L-AMINO ACIDS USING BACTERIA OF THE
   ENTEROBACTERIACEAE FAMILY
<130> C349-C5252
<150> RU2004130954
   <151> 2004-10-22
<160> 14
<170> PatentIn version 3.1
<210> 1
   <211> 1476
   <212> DNA
   <213> Escherichia coli
<220>
   <221> CDS
   <222> (1).. (1476)
<400> 1
   atg aat acc cag tat aat tcc agt tat ata ttt tcg att acc tta gtc        48
   Met Asn Thr Gln Tyr Asn Ser Ser Tyr Ile Phe Ser Ile Thr Leu Val
   1               5                   10                  15
   gct aca tta ggt ggt tta tta ttt ggc tac gac acc gcc gtt att tcc        96
   Ala Thr Leu Gly Gly Leu Leu Phe Gly Tyr Asp Thr Ala Val Ile Ser
               20                  25                  30
   ggt act gtt gag tca ctc aat acc gtc ttt gtt gct cca caa aac tta       144
   Gly Thr Val Glu Ser Leu Asn Thr Val Phe Val Ala Pro Gln Asn Leu
           35                  40                  45
   agt gaa tcc gct gcc aac tcc ctg tta ggg ttt tgc gtg gcc agc gct       192
   Ser Glu Ser Ala Ala Asn Ser Leu Leu Gly Phe Cys Val Ala Ser Ala
       50                  55                  60
   ctg att ggt tgc atc atc ggc ggt gcc ctc ggt ggt tat tgc agt aac       240
   Leu Ile Gly Cys Ile Ile Gly Gly Ala Leu Gly Gly Tyr Cys Ser Asn
   65 70 75 80
   cgc ttc ggt cgt cgt gat tca ctt aag att gct gct gtc ctg ttt ttt 288
   Arg Phe Gly Arg Arg Asp Ser Leu Lys Ile Ala Ala Val Leu Phe Phe
   85 90 95
   att tct ggt gta ggt tct gcc tgg cca gaa ctt ggt ttt acc tct ata 336
   Ile Ser Gly Val Gly Ser Ala Trp Pro Glu Leu Gly Phe Thr Ser Ile
   100 105 110
   aac ccg gac aac act gtg cct gtt tat ctg gca ggt tat gtc ccg gaa 384
   Asn Pro Asp Asn Thr Val Pro Val Tyr Leu Ala Gly Tyr Val Pro Glu
   115 120 125
   ttt gtt att tat cgc att att ggc ggt att ggc gtt ggt tta gcc tca 432
   Phe Val Ile Tyr Arg Ile Ile Gly Gly Ile Gly Val Gly Leu Ala Ser
   130 135 140
   atg ctc tcg cca atg tat att gcg gaa ctg gct cca gct cat att cgc 480
   Met Leu Ser Pro Met Tyr Ile Ala Glu Leu Ala Pro Ala His Ile Arg
   145 150 155 160
   ggg aaa ctg gtc tct ttt aac cag ttt gcg att att ttc ggg caa ctt 528
   Gly Lys Leu Val Ser Phe Asn Gln Phe Ala Ile Ile Phe Gly Gln Leu
   165 170 175
   tta gtt tac tgc gta aac tat ttt att gcc cgt tcc ggt gat gcc agc 576
   Leu Val Tyr Cys Val Asn Tyr Phe Ile Ala Arg Ser Gly Asp Ala Ser
   180 185 190
   tgg ctg aat act gac ggc tgg cgt tat atg ttt gcc tcg gaa tgt atc 624
   Trp Leu Asn Thr Asp Gly Trp Arg Tyr Met Phe Ala Ser Glu Cys Ile
   195 200 205
   cct gca ctg ctg ttc tta atg ctg ctg tat acc gtg cca gaa agt cct 672
   Pro Ala Leu Leu Phe Leu Met Leu Leu Tyr Thr Val Pro Glu Ser Pro
   210 215 220
   cgc tgg ctg atg tcg cgc ggc aag caa gaa cag gcg gaa ggt atc ctg 720
   Arg Trp Leu Met Ser Arg Gly Lys Gln Glu Gln Ala Glu Gly Ile Leu
   225 230 235 240
   cgc aaa att atg ggc aac acg ctt gca act cag gca gta cag gaa att 768
   Arg Lys Ile Met Gly Asn Thr Leu Ala Thr Gln Ala Val Gln Glu Ile
   245 250 255
   aaa cac tcc ctg gat cat ggc cgc aaa acc ggt ggt cgt ctg ctg atg      816
   Lys His Ser Leu Asp His Gly Arg Lys Thr Gly Gly Arg Leu Leu Met
               260                 265                 270
   ttt ggc gtg ggc gtg att gta atc ggc gta atg ctc tcc atc ttc cag      864
   Phe Gly Val Gly Val Ile Val Ile Gly Val Met Leu Ser Ile Phe Gln
           275                 280                 285
   caa ttt gtc ggc atc aat gtg gtg ctg tac tac gcg ccg gaa gtg ttc      912
   Gln Phe Val Gly Ile Asn Val Val Leu Tyr Tyr Ala Pro Glu Val Phe
       290                 295                 300
   aaa acg ctg ggg gcc agc acg gat atc gcg ctg ttg cag acc att att      960
   Lys Thr Leu Gly Ala Ser Thr Asp Ile Ala Leu Leu Gln Thr Ile Ile
   305                 310                 315                 320
   gtc gga gtt atc aac ctc acc ttc acc gtt ctg gca att atg acg gtg     1008
   Val Gly Val Ile Asn Leu Thr Phe Thr Val Leu Ala Ile Met Thr Val
                   325                 330                 335
   gat aaa ttt ggt cgt aag cca ctg caa att atc ggc gca ctc gga atg     1056
   Asp Lys Phe Gly Arg Lys Pro Leu Gln Ile Ile Gly Ala Leu Gly Met
               340                 345                 350
   gca atc ggt atg ttt agc ctc ggt acc gcg ttt tac act cag gca ccg     1104
   Ala Ile Gly Met Phe Ser Leu Gly Thr Ala Phe Tyr Thr Gln Ala Pro
           355                 360                 365
   ggt att gtg gcg cta ctg tcg atg ctg ttc tat gtt gcc gcc ttt gcc     1152
   Gly Ile Val Ala Leu Leu Ser Met Leu Phe Tyr Val Ala Ala Phe Ala
       370                 375                 380
   atg tcc tgg ggt ccg gta tgc tgg gta ctg ctg tcg gaa atc ttc ccg     1200
   Met Ser Trp Gly Pro Val Cys Trp Val Leu Leu Ser Glu Ile Phe Pro
   385                 390                 395                 400
   aat gct att cgt ggt aaa gcg ctg gca atc gcg gtg gcg gcc cag tgg     1248
   Asn Ala Ile Arg Gly Lys Ala Leu Ala Ile Ala Val Ala Ala Gln Trp
                   405                 410                 415
   ctg gcg aac tac ttc gtc tcc tgg acc ttc ccg atg atg gac aaa aac     1296
   Leu Ala Asn Tyr Phe Val Ser Trp Thr Phe Pro Met Met Asp Lys Asn
               420                 425                 430
   tcc tgg ctg gtg gcc cat ttc cac aac ggt ttc tcc tac tgg att tac      1344
   Ser Trp Leu Val Ala His Phe His Asn Gly Phe Ser Tyr Trp Ile Tyr
           435                 440                 445
   ggt tgt atg ggc gtt ctg gca gca ctg ttt atg tgg aaa ttt gtc ccg      1392
   Gly Cys Met Gly Val Leu Ala Ala Leu Phe Met Trp Lys Phe Val Pro
       450                 455                 460
   gaa acc aaa ggt aaa acc ctt gag gag ctg gaa gcg ctc tgg gaa ccg      1440
   Glu Thr Lys Gly Lys Thr Leu Glu Glu Leu Glu Ala Leu Trp Glu Pro
   465                 470                 475                 480
   gaa acg aag aaa aca caa caa act gct acg ctg taa                      1476
   Glu Thr Lys Lys Thr Gln Gln Thr Ala Thr Leu
                   485                 490
<210> 2
   <211> 491
   <212> PRT
   <213> Escherichia coli
<400> 2
   Met Asn Thr Gln Tyr Asn Ser Ser Tyr Ile Phe Ser Ile Thr Leu Val
   1               5                   10                  15
   Ala Thr Leu Gly Gly Leu Leu Phe Gly Tyr Asp Thr Ala Val Ile Ser
               20                  25                  30
   Gly Thr Val Glu Ser Leu Asn Thr Val Phe Val Ala Pro Gln Asn Leu
           35                  40                  45
   Ser Glu Ser Ala Ala Asn Ser Leu Leu Gly Phe Cys Val Ala Ser Ala
       50                  55                  60
   Leu Ile Gly Cys Ile Ile Gly Gly Ala Leu Gly Gly Tyr Cys Ser Asn
   65                  70                  75                  80
   Arg Phe Gly Arg Arg Asp Ser Leu Lys Ile Ala Ala Val Leu Phe Phe
                   85                  90                  95
   Ile Ser Gly Val Gly Ser Ala Trp Pro Glu Leu Gly Phe Thr Ser Ile
               100                 105                 110
   Asn Pro Asp Asn Thr Val Pro Val Tyr Leu Ala Gly Tyr Val Pro Glu
   115 120 125
   Phe Val Ile Tyr Arg Ile Ile Gly Gly Ile Gly Val Gly Leu Ala Ser
   130 135 140
   Met Leu Ser Pro Met Tyr Ile Ala Glu Leu Ala Pro Ala His Ile Arg
   145 150 155 160
   Gly Lys Leu Val Ser Phe Asn Gln Phe Ala Ile Ile Phe Gly Gln Leu
   165 170 175
   Leu Val Tyr Cys Val Asn Tyr Phe Ile Ala Arg Ser Gly Asp Ala Ser
   180 185 190
   Trp Leu Asn Thr Asp Gly Trp Arg Tyr Met Phe Ala Ser Glu Cys Ile
   195 200 205
   Pro Ala Leu Leu Phe Leu Met Leu Leu Tyr Thr Val Pro Glu Ser Pro
   210 215 220
   Arg Trp Leu Met Ser Arg Gly Lys Gln Glu Gln Ala Glu Gly Ile Leu
   225 230 235 240
   Arg Lys Ile Met Gly Asn Thr Leu Ala Thr Gln Ala Val Gln Glu Ile
   245 250 255
   Lys His Ser Leu Asp His Gly Arg Lys Thr Gly Gly Arg Leu Leu Met
   260 265 270
   Phe Gly Val Gly Val Ile Val Ile Gly Val Met Leu Ser Ile Phe Gln
   275 280 285
   Gln Phe Val Gly Ile Asn Val Val Leu Tyr Tyr Ala Pro Glu Val Phe
   290 295 300
   Lys Thr Leu Gly Ala Ser Thr Asp Ile Ala Leu Leu Gln Thr Ile Ile
   305 310 315 320
   Val Gly Val Ile Asn Leu Thr Phe Thr Val Leu Ala Ile Met Thr Val
   325 330 335
   Asp Lys Phe Gly Arg Lys Pro Leu Gln Ile Ile Gly Ala Leu Gly Met
   340 345 350
   Ala Ile Gly Met Phe Ser Leu Gly Thr Ala Phe Tyr Thr Gln Ala Pro
   355 360 365
   Gly Ile Val Ala Leu Leu Ser Met Leu Phe Tyr Val Ala Ala Phe Ala
   370 375 380
   Met Ser Trp Gly Pro Val Cys Trp Val Leu Leu Ser Glu Ile Phe Pro
   385 390 395 400
   Asn Ala Ile Arg Gly Lys Ala Leu Ala Ile Ala Val Ala Ala Gln Trp
   405 410 415
   Leu Ala Asn Tyr Phe Val Ser Trp Thr Phe Pro Met Met Asp Lys Asn
   420 425 430
   Ser Trp Leu Val Ala His Phe His Asn Gly Phe Ser Tyr Trp Ile Tyr
   435 440 445
   Gly Cys Met Gly Val Leu Ala Ala Leu Phe Met Trp Lys Phe Val Pro
   450 455 460
   Glu Thr Lys Gly Lys Thr Leu Glu Glu Leu Glu Ala Leu Trp Glu Pro
   465 470 475 480
   Glu Thr Lys Lys Thr Gln Gln Thr Ala Thr Leu
   485 490
<210> 3
   <211> 180
   <212> DNA
   <213> Artificial sequence
<220>
   <223> hybrid promoter
<400> 3
   ctagatctct cacctaccaa acaatgcccc cctgcaaaaa ataaattcat aaaaaacata 60
   cagataacca tctgcggtga taaattatct ctggcggtgt tgacaattaa tcatcggctc 120
   gtataatgtg tggaattgtg agcgtcagaa tggtctaagg caggtctgaa tgaataccca 180
<210> 4
   <211> 32
   <212> DNA
   <213> Artificial sequence
<220>
   <223> primer
<400> 4
   gtaagatctc tcatgtttga cagcttatca tc 32
<210> 5
   <211> 52
   <212> DNA
   <213> Artificial sequence
<220>
   <223> primer
<400> 5
   ggcgctccac ggagcgcctt tttttctttc gtctgcctaa gctttctaga cg 52
<210> 6
   <211> 55
   <212> DNA
   <213> Artificial sequence
<220>
   <223> primer
<400> 6
   tgggtattca ttcagacctg ccttagacca ttctgacgct cacaattcca cacat 55
<210> 7
   <211> 19
   <212> DNA
   <213> Artificial sequence
<220>
   <223> primer
<400> 7
   gatcaccatc gtcttcttg 19
<210> 8
   <211> 19
   <212> DNA
   <213> Artificial sequence
<220>
   <223> primer
<400> 8
   gtagcgacta aggtaatcg 19
<210> 9
   <211> 60
   <212> DNA
   <213> Artificial sequence
<220>
   <223> primer
<400> 9
   cacaacacta aacctataag ttggggaaat acaatgtgaa gcctgctttt ttatactaag 60
<210> 10
   <211> 60
   <212> DNA
   <213> Artificial sequence
<220>
   <223> primer
<400> 10
   gccgatgggc gccatttttc actgcggcaa gaattacgct caagttagta taaaaaagct 60
<210> 11
   <211> 22
   <212> DNA
   <213> Artificial sequence
<220>
   <223> primer
<400> 11
   tcctggcatt gattcagcct gt 22
<210> 12
   <211> 21
   <212> DNA
   <213> Artificial sequence
<220>
   <223> primer
<400> 12
   ccagcagcat gagagcgatg a 21
<210> 13
   <211> 39
   <212> DNA
   <213> Artificial sequence
<220>
   <223> primer
<400> 13
   cccgaattcg gacaggaaga ttacagcgta gcagtttgt 39
<210> 14
   <211> 39
   <212> DNA
   <213> Artificial sequence
<220>
   <223> primer
<400> 14
   cccgtcgacg atcagaatgg tctaaggcag gtctgaatg 39

## Description

### Technical field

The present invention relates to a method for producing an L-amino acid by fermentation, and more specifically to genes which aid in this fermentation. These genes are useful for the improvement of L-amino acid production, for example, for production of L-threonine, L-lysine, L-histidine, L-phenylalanine, L-arginine and L-glutamic acid.

### Background art

Conventionally, L-amino acids are industrially produced by fermentation methods utilizing strains of microorganisms obtained from natural sources, or mutants thereof. Typically, the microorganisms are modified to enhance production yields of L-amino acids.

Many techniques to enhance production yields of L-amino acids have been reported, including transformation of microorganisms with recombinant DNA (see, for example, U.S. Patent No. 4,278,765). Other techniques for enhancing production yields include increasing the activities of enzymes involved in amino acid biosynthesis and/or desensitizing the target enzymes to feedback inhibition by the resulting L-amino acid (see, for example, WO 95/16042 or U.S. Patent Nos. 4,346,170, 5,661,012 and 6,040,160).

Strains useful in production of L-threonine by fermentation are known, including strains with increased activities of enzymes involved in L-threonine biosynthesis (U.S. Patent Nos. 5,175,107, 5,661,012, 5,705,371, and 5,939,307; EP 0219027), strains resistant to chemicals such as L-threonine and its analogs (WO 01/14525A1, EP 301572 A2, U.S. Patent No. 5,376,538), strains with target enzymes desensitized to feedback inhibition by the produced L-amino acid or its by-products (U.S. Patent Nos. 5,175,107 and 5,661,012), and strains with inactivated threonine degradation enzymes (U.S. Patent Nos. 5,939,307 and 6,297,031).

The known threonine-producing strain VKPM B-3996 (U.S. Patent Nos. 5,175,107 and 5,705,371) is presently one of the best known threonine producers. For construction of the strain VKPM B-3996, several mutations and a plasmid, described below, were introduced into the parent strain *E. coli* K-12 (VKPM B-7). Mutant *thrA* gene (mutation *thrA*442) encodes aspartokinase homoserine dehydrogenase I, which is resistant to feedback inhibition by threonine. Mutant *ilvA* gene (mutation *ilvA442)* encodes threonine deaminase having decreased activity which results in a decreased rate of isoleucine biosynthesis and to a leaky phenotype of isoleucine starvation. In bacteria containing the *ilvA442* mutation, transcription of the *thrABC* operon is not repressed by isoleucine, and therefore is very efficient for threonine production. Inactivation of the *tdh* gene encoding threonine dehydrogenase results in prevention of threonine degradation. The genetic determinant of saccharose assimilation *(scrKYABR* genes) was transferred to said strain. To increase expression of the genes controlling threonine biosynthesis, plasmid pVIC40 containing the mutant threonine operon *thrA442BC* was introduced in the intermediate strain TDH6. The amount of L-threonine accumulated during fermentation of the strain can be up to 85 g/l.

By optimizing the main biosynthetic pathway of a desired compound, further improvement of L-amino acid producing strains can be accomplished via supplementation of the bacterium with increasing amounts of sugars as a carbon source, for example, glucose. Despite the efficiency of glucose transport by PTS, access to the carbon source in a highly productive strain still may be insufficient.

It is known that active transport of sugars and other metabolites into bacterial cells is accomplished by several transport systems. Among these, the XylE protein from *E. coli* is a D-xylose permease, one of two systems in *E. coli* responsible for the uptake of D-xylose; the other being the ATP-dependent ABC transporter XylFGH. The cloned *xylE* gene has been shown to complement *xylE* mutants *in vivo* (Davis, E.O. and Henderson, P.J., J. Biol. Chem., 262(29); 13928-32 (1987)). The XylE-mediated transport in whole cells is inhibited by protonophores and elicits an alkaline pH change (Lam, V.M. et al, J. Bacteriol. 143(1); 396-402 (1980)). Experiments using *xylE and xylF* mutants have established that XylE protein has a K_{M} of 63-169 *µ*M for D-xylose (Sumiya. M. et al, Receptors Channels, 3(2); 117-28 (1995)). The XylE protein is a member of the major facilitator superfamily (MFS) of transporters (Griffith, J.K. et al, Curr. Opin. Cell Biol. 4(4); 684-95 (1992)) and appears to function as a D-xylose/proton symporter. The *xylE* gene probably constitutes a monocistronic operon whose expression is inducible by D-xylose. Imported xylose is catabolized to xylulose-5-phosphate by the action of the XylA (xylose isomerase) and XylB (xylulokinase) enzymes. Under appropriate conditions, the xylose isomerase encoded by the *xylA* gene also efficiently catalyzes the conversion of D-glucose to D-fructose (Wovcha, M.G. et al, Appl Environ Microbiol. 45(4): 1402-4 (1983)).

However, there has been no report to date of using a bacterium of the *Enterobacteriaceae family* having an enhanced activity of D-xylose permease for increasing the production of L-amino acids.

### Disclosure of the Invention

An object of present invention is to enhance the productivity of L-amino acid-producing strains and to provide a method for producing non-aromatic or aromatic L-amino acids using these strains.

This aim was achieved by finding that the increasing the expression of the *xylE* gene encoding D-xylose permease enhances production of L-amino acids, such as L-threonine, L-lysine, L-histidine, L-phenylalanine, L-arginine and L-glutamic acid. Thus the present invention has been completed.

The present invention uses an L-amino acid-producing bacterium of the *Enterobacteriaceae* family, wherein said bacterium has been modified to enhance an activity of D-xylose permease.

The present invention uses the bacterium described above, wherein said activity of said D-xylose permease is enhanced by increasing the expression of a gene which encodes D-xylose permease.

The present invention uses the bacterium described above, wherein said activity of D-xylose permease is enhanced by modifying an expression control sequence of the gene encoding D-xylose permease so that the gene expression is enhanced or by increasing the copy number of the gene encoding D-xylose permease.

The present invention uses the bacterium described above, wherein said bacterium has been additionally modified to enhance an activity of glucokinase.

JP2004-254694 concerns method for production of L-amino acids by fermentation of glucose and pentose mixture.

The present invention uses the bacterium described above, wherein said bacterium has been additionally modified to enhance an activity of xylose isomerase.

The present invention uses the bacterium described above, wherein said bacterium has been modified to increase the expression of the *xylABFGHR* locus.

The present invention uses the bacterium described above, wherein the bacterium is selected from the group consisting of the genera *Escherichia, Enterobacter, Erwinia, Klebsiella, Pantoea, Providencia, Salmonella, Serratia, Shigella,* and *Morganella.*

The present invention uses the bacterium described above,
wherein said gene encodes a D-xylose permease selected from the group consisting of:
(A) a protein which comprises the amino acid sequence of SEQ ID NO: 2; and
(B) a variant protein of the amino acid sequence shown in SEQ ID NO: 2 which has an activity of D-xylose permease.

The present invention uses the bacterium described above,
wherein said gene encoding D-xylose permease comprises a DNA selected from the group consisting of:
(a) a DNA which comprises a nucleotide sequence of nucleotides 1 to 1476 in SEQ ID NO: 1; and
(b) a DNA which is hybridizable with a nucleotide sequence of nucleotides 1-1476 in SEQ ID NO: 1, or a probe which can be prepared from said nucleotide sequence under stringent conditions, and encodes a protein having an activity of D-xylose permease.

The present invention uses the bacterium described above,
wherein said stringent conditions comprise those in which washing is performed at 60°C at a salt concentration of 1 x SSC and 0.1 % SDS for 15 minutes.

The present invention uses the bacterium described above,
wherein said bacterium is an L-threonine producing bacterium.

The present invention uses the bacterium described above,
wherein said bacterium has been additionally modified to enhance expression of a gene selected from the group consisting of
- the mutant *thrA* gene which codes for aspartokinase homoserine dehydrogenase I and is resistant to feedback inhibition by threonine,
- the *thrB* gene which codes for homoserine kinase,
- the *thrC* gene which codes for threonine synthase,
- the *rhtA* gene which codes for a putative transmembrane protein, and
- any combination thereof.

The present invention uses the bacterium described above, wherein said bacterium has been modified to increase expression of said mutant *thrA* gene, said *thrB* gene, *said thrC* gene, and said *rhtA* gene.

The present invention uses the bacterium described above, wherein said bacterium is an L-lysine producing bacterium.

The present invention uses the bacterium described above, wherein said bacterium is an L-histidine producing bacterium.

The present invention uses the bacterium described above, wherein said bacterium is an L-phenylalanine producing bacterium.

The present invention uses the bacterium described above, wherein said bacterium is an L-arginine producing bacterium.

The present invention uses the bacterium described above, wherein said bacterium is an L-glutamic acid producing bacterium.

It is an object of the present invention to provide a method for producing an L-amino acid which comprises cultivating the bacterium described above in a culture medium containing glucose, allowing accumulation of the L-amino acid in the culture medium, and isolating the L-amino acid from the culture medium.

The present invention uses the method described above,
wherein the culture medium contains xylose.

The present invention uses the method described above, wherein said L-amino acid is L-threonine.

It is a further object of the present invention to provide the method described above, wherein said L-amino acid is L-lysine.

It is a further object of the present invention to provide the method described above, wherein said L-amino acid is L-histidine.

It is a further object of the present invention to provide the method described above, wherein said L-amino acid is L-phenylalanine.

It is a further object of the present invention to provide the method described above, wherein said L-amino acid is L-arginine.

It is a further object of the present invention to provide the method described above, wherein said L-amino acid is L-glutamic acid.

### Brief Description of the Drawings

Figure 1 shows the structure of the region upstream of the *xylE* gene in the chromosome *of E. coli* and the structure of an integrated DNA fragment containing the *cat* gene and a hybrid P_{L-tac} promoter.
Figure 2 shows growth curves *of E. coli* strains MG1655, MG1655 Δ*ptsHI-crr* and MG1655P_{L-tac}xylE grown on medium with glucose. Legend: MG = *E. coli* MG1655; MG Δpts = *E. coli* MG1655 Δ*ptsHI-crr;* MG Δpts P xylE = *E. coli* MG1655 Δ*ptsHI-crr* P_{L-tac}xylE.

### Best Mode for Carrying Out the Invention

In the present invention, "L-amino acid-producing bacterium" means a bacterium which has an ability to produce and excrete an L-amino acid in a medium, when the bacterium is cultured in the medium. The L-amino acid-producing ability may be imparted or enhanced by breeding. The term "L-amino acid-producing bacterium" as used herein also means a bacterium which is able to produce and cause accumulation of an L-amino acid in a culture medium in an amount larger than a wild-type or parental strain *of E. coli,* such as *E. coli* K-12, and preferably means that the bacterium is able to cause accumulation in a medium of an amount not less than 0.5 g/L, more preferably not less than 1.0 g/L of the target L-amino acid. The term "L-amino acids" include L-alanine, L-arginine, L-asparagine, L-aspartic acid, L-cysteine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, and L-valine. L-threonine, L-lysine, L-histidine, L-phenylalanine, L-arginine, and L-glutamic acid are particularly preferred.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella , Morganella, Yersinia,* etc. Specifically, those classified into the *Enterobacteriaceae* according to the taxonomy used in the NCBI (National Center for Biotechnology Information) database
(http://www.ncbi.nlm.nih.gov/htbinpost/Taxonomy/wgetorg?mode=Tree&id=1236&lvl=3&kee p=1&srchmode=1&unlock can be used. A bacterium belonging to the genus of *Escherichia* or *Pantoea* is preferred.

The phrase "a bacterium belonging to the genus *Escherichia"* means that the bacterium is classified into the genus *Escherichia* according to the classification known to a person skilled in the art of microbiology. Examples of a microorganism belonging to the genus *Escherichia* as used in the present invention include, but are not limited to, *Escherichia coli* (*E. coli*).

The bacterium belonging to the genus *Escherichia* that can be used in the present invention is not particularly limited; however, e.g., bacteria described by Neidhardt, F.C. et al. (*Escherichia coli* and *Salmonella typhimurium,* American Society for Microbiology, Washington D.C., 1208, Table 1) are encompassed by the present invention.

The term "a bacterium belonging to the genus *Pantoea"* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like, based on the nucleotide sequence analysis of 16S rRNA etc. (Int. J. Syst. Bacteriol., 43,162-173 (1993)).

The bacterium used in the present invention encompasses a strain of the *Enterobacteriaceae* family which has an ability to produce an L-amino acid and has been modified to increase the expression of xylE gene encoding D-xylose permease. In addition, the bacterium used in the present invention encompasses a strain of the *Enterobacteriaceae* family which has an ability to produce a L-amino acid and does not have a native activity of D-xylose permease, and has been transformed with a DNA fragment encoding D-xylose permease.

The phrase "activity of D-xylose permease" means an activity of transporting sugars, such as xylose and glucose, into the cell. Activity of D-xylose permease can be detected by complementation of growth delay of the bacterium which has a disrupted PTS-system of sugar transport (see, for example, the *AptsHI-crr* mutant described in the Examples) or by complementation *xylE* mutations *in vivo* (Davis, E.O. and Henderson, P.J., J. Biol. Chem., 262(29); 13928-32 (1987)).

The phrase "bacterium has been modified to enhance an activity of D-xylose permease" means that the activity per cell is higher than that of a non-modified strain, for example, a wild-type strain. Examples of such modifications include increasing the number of D-xylose permease molecules per cell, increasing the specific activity per D-xylose permease molecule, and so forth. Furthermore, a wild-type strain that may be used for comparison purposes includes, for example, *Escherichia coli* K-12. In the present invention, the amount of the accumulated L-amino acid, for example, L-threonine or L-arginine, can be increased in a culture medium as a result of enhancing the intracellular activity of D-xylose permease.

Enhancement of D-xylose permease activity in a bacterial cell can be attained by increasing the expression of the *xylE* gene encoding D-xylose permease. Any *xylE* gene derived from bacteria belonging to the genus *Escherichia,* as well as any *xylE* gene derived from other bacteria, such as coryneform bacteria, may be used as the D-xylose permease gene in the present invention. The *xylE* genes derived from bacteria belonging to the genus *Escherichia* are preferred.

The phrase "increasing the expression of the gene" means that the expression amount of the gene is higher than that of a non-modified strain, for example, a wild-type strain. Examples of such modification include increasing the copy number of gene(s) per cell, increasing the expression level of the gene(s), and so forth. The quantity of the copy number of a gene is measured, for example, by restricting the chromosomal DNA followed by Southern blotting using a probe based on the gene sequence, fluorescence *in situ* hybridization (FISH), and the like. The level of gene expression can be measured by various methods including Northern blotting, quantitative RT-PCR, and the like. Furthermore, a wild-type strain that can act as a control includes, for example, *Escherichia coli* K-12 or *Pantoea ananatis* FERM BP-6614 (US2004180404A1). *Pantoea ananatis* FERM BP-6614 was depsited at the National Institute of Bioscience and Human-Technology, Agency of Industrial Science and Technology, Ministry of International Trade and Industry (currently, International Patent Organism Depositary, National Institute of Advanced Industrial Science and Technology) on February 19,1998 and received an accession number of FERM P-16644. It was then converted to an international deposit under the provisions of Budapest Treaty on January 11,1999 and received an accession number of FERM BP-6614. Although this strain was identified as *Enterobacter agglomerans* when it was isolated, it has been re-classified into *Pantoea ananatis* based on nucleotide sequence analysis of 16S rRNA etc. as described above.

As a result of enhancing the intracellular activity of D-xylose permease, L-amino acid accumulation, for example L-threonine, L-lysine, L-histidine, L-phenylalanine or L-glutamic acid accumulation in a medium is increased.

The *xylE* gene which encodes D-xylose permease, namely D-xylose/proton symporter, from *Escherichia coli* has been elucidated (nucleotide numbers 4240277 to 4238802 in the sequence of GenBank accession NC_000913.2, gi:49175990). The *xylE* gene is located between the *yjbA* ORF and the *malG* gene on the chromosome of *E. coli* K-12. The other *xylE* genes which encodes D-xylose permease have also been elucidated (AAN45595. xylose-proton sym...[gi:24054686], AAM41050. MFS transporter...[gi:21112853]: *Xanthomonas campestris:* XCC1759). In the present invention, the *xylE* gene from *Escherichia coli* is represented by SEQ ID NO. 1.

Upon being transported into the cell, glucose is phosphorylated by glucokinase, which is encoded by the *glk* gene. So, it is also desirable to modify the bacterium to have enhanced activity of glucokinase. The *glk* gene which encodes glucokinase of *Escherichia coli* has been elucidated (nucleotide numbers 2506481 to 2507446 in the sequence of GenBank accession NC_000913.1, gi:16127994). The *glk* gene is located between the *b2387* and the *b2389* ORFs on the chromosome *of E. coli* K-12.

Under appropriate conditions, the xylose isomerase encoded by the *xylA* gene also efficiently catalyzes the conversion of D-glucose to D-fructose (Wovcha, M.G. et al, Appl Environ Microbiol. 45(4): 1402-4 (1983)). So, it is also desirable to modify the bacterium to have an enhanced activity of xylose isomerase. The *xylA* gene which encodes xylose isomerase of *Escherichia coli* has been elucidated (nucleotide numbers 3728788 to 3727466 in the sequence of GenBank accession NC_000913.2, gi: 49175990). The *xylA* gene is located between *the xylB and xylF* genes on the chromosome of *E*. *coli* K-12.

When the culture medium contains xylose as an additional carbon source, increasing the activity of the xylose utilization enzymes is necessary. The "xylose utilization enzymes" include enzymes of xylose transport, xylose isomerization and xylose phosphorylation, and regulatory proteins. Such enzymes include xylose isomerase, xylulokinase, xylose transporters, and xylose transcriptional activator. Xylose isomerase catalyzes the reaction of isomerization of D-xylose to D-xylulose. Xylulokinase catalyzes the reaction of phosphorylation of D-xylulose using ATP yielding D-xylulose-5-phosphate and ADP. The presence of activity of xylose utilization enzymes, such as xylose isomerase and xylulokinase, is determined by complementation of corresponding xylose isomerase-negative or xylulokinase-negative *E. coli* mutants, respectively.

Genes coding for the above mentioned xylose utilization enzymes are located in the *xylABFGHR* locus on the chromosome of *Escherichia coli.* The gene coding for xylulokinase (EC numbers 2.7.1.17) is known and has been designated *xylB* (nucleotide numbers 3725546 to 3727000 in the sequence of GenBank accession NC_000913.1, gi:16131435). The gene coding for the xylose binding protein transport system is known and has been designated *xylF* (nucleotide numbers 3728760 to 3729752 in the sequence of GenBank accession NC_000913.1, gi:16131437). The gene coding for the putative ATP-binding protein of the xylose transport system is known and has been designated *xylG* (nucleotide numbers 3729830 to3731371 in the sequence of GenBank accession NC_000913.1, gi:16131438). The gene coding for the permease component of the ABC-type xylose transport system is known and has been designated *xylH* (nucleotide numbers 3731349 to 3732530 in the sequence of GenBank accession NC_000913.1, gi:16131439). The gene coding for the transcriptional regulator of the *xyl* operon is known and has been designated *xylR* (nucleotide numbers 3732608 to 3733786 in the sequence of GenBank accession NC_000913.1, gi:16131440).

Therefore, *xylE, glk* and genes of the *xylABFGHR* locus can be obtained by PCR (polymerase chain reaction; refer to White, T.J. et al., Trends Genet., 5, 185 (1989)) utilizing primers prepared based on the known nucleotide sequence of the gene. Genes coding for D-xylose permease of other microorganisms can be obtained in a similar manner.

The *xylE* gene derived from *Escherichia coli* is exemplified by a DNA which encodes the following protein (A) or (B):
(A) a protein which has the amino acid sequence shown in SEQ ID NO: 2; or
(B) a variant protein of the amino acid sequence shown in SEQ ID NO: 2, which has an activity of D-xylose permease.

The phrase "variant protein" as used in the present invention means a protein which has changes in the sequence, whether they are deletions, insertions, additions, or substitutions of amino acids, but still maintains the desired activity at a useful level, for example, useful for the enhanced production of an L-amino acid. The number of changes in the variant protein depends on the position or the type of amino acid residues in the three dimensional structure of the protein. It may be 2 to 30, preferably 2 to 15, and more preferably 2 to 5 for the protein (A). These changes in the variants can occur in regions of the protein which are not critical for the function of the protein. This is because some amino acids have high homology to one another so the three dimensional structure or activity is not affected by such a change. These changes in the variant protein can occur in regions of the protein which are not critical for the function of the protein. Therefore, the protein variant (B) may be one which has a homology of not less than 70 %, preferably 80 %, and more preferably 90 %, and most preferably 95 % with respect to the entire amino acid sequence of D-xylose permease shown in SEQ ID NO. 2, as long as the activity of D-xylose permease is maintained. Homology between two amino acid sequences can be determined using the well-known methods, for example, the computer program BLAST 2.0, which calculates three parameters: score, identity and similarity.

The DNA, which encodes substantially the same protein as the D-xylose permease described above, may be obtained, for example, by modifying the nucleotide sequence of DNA encoding D-xylose permease (SEQ ID NO: 1), for example, by means of the site-directed mutagenesis method so that one or more amino acid residues at a specified site involve deletion, substitution, insertion, or addition. A DNA modified as described above may be obtained by conventionally known mutation treatments. Such treatments include hydroxylamine treatment of the DNA encoding proteins of the present invention, or treatment of the bacterium containing the DNA with UV irradiation or a reagent such as N-methyl-N'-nitro-N-nitrosoguanidine or nitrous acid.

The substitution, deletion, insertion or addition of one or several amino acid residues should be conservative mutation(s) so that the activity is maintained. The representative conservative mutation is a conservative substitution. Examples of conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val.

A DNA encoding substantially the same protein as D-xylose permease can be obtained by expressing a DNA having the mutation as described above in an appropriate cell, and investigating the activity of any expressed product. A DNA encoding substantially the same protein as D-xylose permease can also be obtained by isolating a DNA, that is hybridizable with a probe having a nucleotide sequence which contains, for example, the nucleotide sequence shown as SEQ ID NO: 1, under the stringent conditions, and encodes a protein having the D-xylose permease activity. The "stringent conditions" referred to herein are conditions under which so-called specific hybrids are formed, and non-specific hybrids are not formed. For example, stringent conditions can be exemplified by conditions under which DNAs having high homology, for example, DNAs having homology of not less than 50%, preferably 80 %, and still more preferably 90 %, and most preferably 95 % are able to hybridize with each other, but DNAs having homology lower than the above are not able to hybridize with each other. Alternatively, stringent conditions may be exemplified by conditions under which DNA is able to hybridize at a salt concentration equivalent to ordinary washing conditions in Southern hybridization, i.e., 1 x SSC, 0.1% SDS, preferably 0.1 x SSC, 0.1% SDS, at 60°C. Duration of washing depends on the type of membrane used for blotting and, as a rule, what is recommended by the manufacturer. For example, recommended duration of washing the Hybond™ N+ nylon membrane (Amersham) under stringent conditions is 15 minutes. Preferably, washing may be performed 2 to 3 times.

A partial sequence of the nucleotide sequence of SEQ ID NO: 1 can also be used as a probe. Probes may be prepared by PCR using primers based on the nucleotide sequence of SEQ ID NO: 1, and a DNA fragment containing the nucleotide sequence of SEQ ID NO: 1 as a template. When a DNA fragment having a length of about 300 bp is used as the probe, the hybridization conditions for washing include, for example, 50°C, 2 x SSC and 0.1% SDS.

The substitution, deletion, insertion, or addition of nucleotides as described above also includes mutation which naturally occurs (mutant or variant), for example, due to variety in the species or genus of bacteria, and which contains the D-xylose permease.

"Transformation of a bacterium with DNA encoding a protein" means introduction of the DNA into a bacterium, for example, by conventional methods. Transformation of this DNA will result in an increase in expression of the gene encoding the protein of the present invention, and will enhance the activity of the protein in the bacterial cell. Methods of transformation include any known methods that have hitherto been reported. For example, a method of treating recipient cells with calcium chloride so as to increase permeability of the cells to DNA has been reported for *Escherichia coli* K-12 (Mandel, M. and Higa, A., J. Mol. Biol., 53, 159 (1970)) and may be used.

Methods of gene expression enhancement include increasing the gene copy number. Introducing a gene into a vector that is able to function in a bacterium of the *Enterobacteriaceae* family increases the copy number of the gene. Preferably, low copy vectors are used. Examples of low-copy vectors include, but are not limited to, pSC101, pMW118, pMW119, and the like. The term "low copy vector" is used for vectors, the copy number of which is up to 5 copies per cell.

Enhancement of gene expression may also be achieved by introduction of multiple copies of the gene into a bacterial chromosome by, for example, a method of homologous recombination, Mu integration or the like. For example, one act of Mu integration allows introduction of up to 3 copies of the gene into a bacterial chromosome.

Increasing the copy number of the D-xylose permease gene can also be achieved by introducing multiple copies of the D-xylose permease gene into the chromosomal DNA of the bacterium. In order to introduce multiple copies of the gene into a bacterial chromosome, homologous recombination is carried out using a sequence whose multiple copies exist as targets in the chromosomal DNA. Sequences having multiple copies in the chromosomal DNA include, but are not limited to, repetitive DNA, or inverted repeats existing at the end of a transposable element. Also, as disclosed in U.S. Patent No. 5,595,889, it is possible to incorporate the D-xylose permease gene into a transposon, and allow it to be transferred to introduce multiple copies of the gene into the chromosomal DNA.

Enhancement of gene expression may also be achieved by placing the DNA of the present invention under the control of a potent promoter. For example, the *lac* promoter, the *trp* promoter, the *trc* promoter, and the P_{R} or the P_{L} promoter of lambda phage are known as potent promoters. Enhancement of gene expression may also be achieved by placing a potent terminator downstream of the DNA of the present invention. Use of a potent promoter and/or terminator can be combined with multiplication of gene copies. Alternatively, the effect of a promoter can be enhanced by, for example, introducing a mutation into the promoter to increase the transcription level of a structural gene (coding region of a gene) located downstream of the promoter. Similarly, the effect of a terminator can be enhanced by, for example, introducing a mutation into the terminator to increase the turnover of transcription of a gene located upstream of the terminator.

Furthermore, it is known that substitution of several nucleotides in the spacer between ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the *rhtA23* mutation is an A-for-G substitution at the -1 position relative to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances the *rhtA* gene expression and, as a consequence, increases the resistance to threonine, homoserine and some other substances transported out of cells.

Moreover, it is also possible to introduce a nucleotide substitution into a promoter or terminator region of the D-xylose permease gene on the bacterial chromosome, which results in a stronger promoter or terminator function. The alteration of the expression control sequence can be performed, for example, in the same manner as the gene substitution using a temperature-sensitive plasmid, as disclosed in International Patent Publication WO 00/18935 and Japanese Patent Application Laid-Open No. 1-215280.

Methods for preparation of plasmid DNA include, but are not limited to, digestion and ligation of DNA, transformation, selection of an oligonucleotide as a primer and the like, or other methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

The bacterium used in the present invention can be obtained by introduction of the aforementioned DNAs into a bacterium which inherently has the ability to produce an L-amino acid. Alternatively, the bacterium used in the present invention can be obtained by imparting an
ability to produce an L-amino acid to a bacterium already containing the DNAs.

### L-threonine producing bacteria

Examples of parent strains for deriving the L-threonine-producing bacteria used in the present invention include, but are not limited to, L-threonine-producing bacteria belonging to the genus *Escherichia,* such as *E. coli* TDH-6/pVIC40 (VKPM B-3996) (U.S. Patent Nos. 5,175,107 and 5,705,371), *E. coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 1978,14: 947-956), *E. coli* VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain B-3996 contains the plasmid pVIC40 which was obtained by inserting a *thrA*BC* operon which includes a mutant *thrA* gene into a RSF1010-derived vector. This mutant *thrA* gene encodes aspartokinase homoserine dehydrogenase I which has substantially desensitized feedback inhibition by threonine. The strain B-3996 was deposited in the All-Union Scientific Center of Antibiotics (Nagatinskaya Street 3-A, 117105 Moscow, Russian Federation) on November 19, 1987 under accession number RIA 1867. The strain was also deposited in the Russian National Collection of Industrial Microorganisms (VKPM; Dorozhny proezd. 1, Moscow 117545, Russian Federation) on April 7, 1987 under accession number B-3996.

Preferably, the bacterium used in the present invention is additionally modified to enhance
expression of one or more of the following genes:
- the mutant *thrA* gene which encodes aspartokinase homoserine dehydrogenase I resistant to feedback inhibition by threonine;
- the *thrB* gene which encodes homoserine kinase;
- the *thrC* gene which encodes threonine synthase;
- the *rhtA* gene which encodes a putative transmembrane protein;
- the *asd* gene which encodes aspartate-ß-semialdehyde dehydrogenase; and
- the *aspC* gene which encodes aspartate aminotransferase (aspartate transaminase);

The *thrA* gene which encodes aspartokinase homoserine dehydrogenase I of *Escherichia coli* has been elucidated (nucleotide positions 337 to 2799, GenBank accession no. NC_000913.2, gi: 49175990). The *thrA* gene is located between the *thrL* and *thrB* genes on the chromosome *of E. coli* K-12. The *thrB* gene which encodes homoserine kinase of *Escherichia coli* has been elucidated (nucleotide positions 2801 to 3733, GenBank accession no. NC_000913.2, gi: 49175990). The *thrB* gene is located between *thrA* and *thrC* genes on the chromosome of *E. coli* K-12. The *thrC* gene which encodes threonine synthase of *Escherichia coli* has been elucidated (nucleotide positions 3734 to 5020, GenBank accession no. NC_000913.2, gi: 49175990). The *thrC* gene is located between the *thrB* gene and the *yaaX* open reading frame on the chromosome of *E*. *coli* K-12. All three genes function as a single threonine operon.

A mutant *thrA* gene which encodes aspartokinase homoserine dehydrogenase I resistant to feedback inhibition by threonine, as well as the *thrB* and *thrC* genes, can be obtained as one operon from the well-known plasmid pVIC40 which is present in the threonine producing *E*. *coli* VKPM B-3996. Plasmid pVIC40 is described in detail in U.S. Patent No. 5,705,371.

The *rhtA* gene exists at 18 min on the *E. coli* chromosome close to the *glnHPQ* operon, which encodes components of the glutamine transport system. The *rhtA* gene is identical to ORF1 (*ybiF* gene, positions 764 to 1651, GenBank accession no. AAA218541, gi:440181) and located between the *pexB* and *ompX* genes. The unit expressing a protein encoded by the ORF1 has been designated the *rhtA* gene (rht: resistance to homoserine and threonine). Also, it was revealed that the *rhtA23* mutation is an A-for-G substitution at position -1 with respect to the ATG start codon (ABSTRACTS of the 17th International Congress of Biochemistry and Molecular Biology in conjugation with the Annual Meeting of the American Society for Biochemistry and Molecular Biology, San Francisco, California, August 24-29,1997, abstract No. 457, EP 1013765 A).

The *asd* gene *of E. coli* has already been elucidated (nucleotide positions 3572511 to 3571408, GenBank accession no. NC_000913.1, gi:16131307), and can be obtained by PCR (polymerase chain reaction; refer to White, T.J. et al., Trends Genet., 1989, 5:185), utilizing primers based on the nucleotide sequence of the gene. The *asd* genes of other microorganisms can be obtained in a similar manner.

Also, the *aspC* gene *of E. coli* has already been elucidated (nucleotide positions 983742 to 984932, GenBank accession no. NC_000913.1, gi:16128895), and can be obtained by PCR. The *aspC* genes of other microorganisms can be obtained in a similar manner.

### L-lysine producing bacteria

Examples of L-lysine producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, a-chlorocaprolactam and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine producing bacterium of *Escherichia coli.* This bacterial strain was bred by conferring AEC resistance to the strain W3110, which was derived from *Escherichia coli* K-12. The resulting strain was designated as the *Escherichia coli* AJ13069 strain, and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6,1994 and received an accession number of FERM P-14690. Then, it was converted to an international deposit under the provisions of the Budapest Treaty on September 29,1995, and received an accession number of FERM BP-5252 (U.S. Patent No. 5,827,698).

### L-histidine producing bacteria

Examples of parent strains for deriving the L-histidine-producing bacteria used in the present invention include, but are not limited to, L-histidine-producing bacteria belonging to the genus *Escherichia,* such as *E. coli* strain 24 (VKPM B-5945, RU2003677); *E. coli* strain 80 (VKPM B-7270, RU2119536); *E. coli* NRRL B-12116 - B12121 (U.S. Patent No. 4,388,405); *E. coli* H-9342 (FERM BP-6675) and H-9343 (FERM BP-6676) (U.S. Patent No. 6,344,347); *E. coli* H-9341 (FERM BP-6674) (EP 1085087); *E. coli* AI80/pFM201 (U.S. Patent No. 6,258,554), and the like.

### L-phenylalanine producing bacteria

Examples of parent strains for deriving the L-phenylalanine-producing bacteria used in the present invention include, but are not limited to, L-phenylalanine-producing bacteria belonging to the genus *Escherichia,* such as *E. coli* AJ12739 (tyrA::Tn10, tyrR) (VKPM B-8197); *E. coli* HW1089 (ATCC 55371) harboring the pheA34 gene (U.S. Patent No. 5,354,672); *E. coli* MWEC101-b (KR8903681); *E. coli* NRRL B-12141, NRRL B-12145, NRRL B-12146, and NRRL B-12147 (U.S. Patent No. 4,407,952). Also, as a parent strain, *E. coli* K-12 [W3110 (tyrA)/pPHAB (FERM BP-3566), *E. coli* K-12 [W3110 (tyrA)/pPHAD] (FERM BP-12659), *E*. *coli* K-12 [W3110 (tyrA)/pPHATerm] (FERM BP-12662), and *E. coli* K-12 [W3110 (tyrA)/pBR-aroG4, pACMAB] named AJ 12604 (FERM BP-3579) may be used (EP 488424 B1). Furthermore, L-phenylalanine-producing bacteria belonging to the genus *Escherichia* with an enhanced activity of the protein encoded by the *yedA* gene or the *yddG* gene may also be used (U.S. Patent Applications 2003/0148473 A1 and 2003/0157667 A1).

### L-arginine producing bacteria

Examples of parent strains for deriving the L-arginine-producing bacteria used in the present invention include, but are not limited to, L-arginine-producing bacteria, such as *E. coli* strain 237 (VKPM B-7925) (U.S. Patent Application 2002/0058315 A1) and its derivative strains harboring mutant N-acetylglutamate synthase (Russian Patent Application No. 2001112869), *E*. *coli* strain 382 (VKPM B-7926) (EP 1170358 A1), an arginine-producing strain which has the *argA* gene encoding N-acetylglutamate synthetase introduced therein (JP 57-5693A), and the like.

### L-glutamic acid producing bacteria

Examples of parent strains for deriving the L-glutamic acid-producing bacteria used in the present invention include, but are not limited to, L-glutamic acid-producing bacteria belonging to the genus *Escherichia,* such as *E. coli* VL334thrC⁺ (EP 1172433). *E. coli* VL334 (VKPM B-1641) is an L-isoleucine and L-threonine auxotrophic strain having mutations in the *thrC* and *ilvA* genes (U.S. Patent No. 4,278,765). A wild-type allele of the *thrC* gene was transferred by the method of general transduction, using bacteriophage P1 grown on wild-type *E. coli* K12 (VKPM B-7) cells. As a result, an L-isoleucine auxotrophic strain VL334thrC⁺ (VKPM B-8961) was obtained. This strain is able to produce L-glutamic acid.

Examples of parent strains for deriving the L-glutamic acid-producing bacteria used in the present invention include, but are not limited to, mutants which are deficient in a-ketoglutarate dehydrogenase activity or mutants which have a reduced a-ketoglutarate dehydrogenase activity. Bacteria belonging to the genus *Escherichia* deficient in a-ketoglutarate dehydrogenase activity or having a reduced a-ketoglutarate dehydrogenase activity and methods for obtaining them are described in U.S. Patent Nos. 5,378,616 and 5,573,945. Specifically, these strains include the following:
*E. coli* W3110sucA::Kmr
*E. coli* AJ12624 (FERM BP-3853)
*E. coli* AJ12628 (FERM BP-3854)
*E. coli* AJ12949 (FERM BP-4881)
*E. coli* W3110sucA::Kmr is obtained by disrupting the α-ketoglutarate dehydrogenase gene (hereinafter referred to as *"sucA* gene") of E. coli W3110. This strain is completely deficient in α-ketoglutarate dehydrogenase.

Other examples of L-glutamic acid-producing bacteria include mutant strains belonging to the genus *Pantoea* which are deficient in α-ketoglutarate dehydrogenase activity or have a decreased α-ketoglutarate dehydrogenase activity, and can be obtained as described above. Such strains include *Pantoea ananatis* AJ13356. (U.S. Patent No. 6,331,419). *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6,1-1, Higashi 1-Chome, Tsukuba-shi, Ibaraki-ken, 305-8566, Japan) on February 19,1998 under accession no. FERM P-16645. It was then converted to an international deposit under the provisions of the Budapest Treaty on January 11,1999 and received accession no. FERM BP-6615. *Pantoea ananatis* AJ13356 is deficient in a-ketoglutarate dehydrogenase activity as a result of disruption of the aKGDH-E1 subunit gene *(sucA).* The above strain was identified as *Enterobacter agglomerans* when it was isolated and deposited as *Enterobacter agglomerans* AJ13356. However, it was recently re-classified as *Pantoea ananatis* on the basis of nucleotide sequencing of 16S rRNA and so forth. Although AJ13356 was deposited at the aforementioned depository as *Enterobacter agglomerans,* for the purposes of this specification, they are described as *Pantoea ananatis.*

### Production of L-amino acids

Oxaloacetate (OAA) serves as a substrate for the reaction which results in synthesis of Thr and Lys. OAA results from a reaction of PEP with phosphoenol pyrvate carboxlase (PEPC) functioning as a catalyst. Therefore, elevation of the PEPC concentration in a cell can be very important for fermentative production of these amino acids. When using glucose as the carbon source in fermentation, glucose is internalized by the glucose-phosphontransferase (Glc-PTS) system. This system consumes PEP, and proteins in the PTS are encoded by *ptsG* and *ptsHIcrr.* During internalization, one molecule of PEP and one molecule of pyruvate (Pyr) are generated from one molecule of glucose.

An L-threonine-producing strain and an L-lysine-producing strain which have been modified to have an ability to utilize sucrose (Scr-PTS) have higher productivity of these amino acids when cultured in sucrose rather than glucose (EP 1149911 A2). It is believed that three molecules of PEP and one molecule of Pyr are generated from one molecule of sucrose by the Scr-PTS, increasing the ratio of PEP/Pyr, and thereby facilitating the synthesis of Thr and Lys from sucrose. Furthermore, it has been reported that Glc-PTS is subject to several expression controls (Postma P.W. et al., Microbiol Rev., 57(3), 543-94 (1993); Clark B. et al. J. Gen. Microbiol., 96(2), 191-201 (1976); Plumbridge J., Curr. Opin. Microbiol., 5(2),187-93 (2002); Ryu S. et al., J. Biol. Chem., 270(6):2489-96 (1995)), and hence it is possible that the incorporation of glucose itself can be a rate-limiting step in amino acid fermentation.

Increasing the ratio of PEP/Pyr even more by increasing expression of the *xylE* gene in a threonine-producing strain, a lysine-producing strain, a histidine-producing strain, a phenylalanine-producing strain and/or a glutamic acid -producing strain should further increase amino acid production. Because four molecules of PEP are generated from two molecules of glucose, the ratio of PEP/Pyr is expected to be greatly improved. Due to the increased expression of the *xylE* gene, removal of the expression control of glc-PTS is expected.

The method for producing an L-amino acid of the present invention includes the steps of cultivating the bacterium used in the present invention in a culture medium, allowing the L-amino acid to accumulate in the culture medium, and collecting the L-amino acid from the culture medium. Furthermore, the method of the present invention includes a method for producing L-threonine, L-lysine, L-histidine, L-phenylalanine, L-arginine or L-glutamic acid, including the steps of cultivating the bacterium used in the present invention in a culture medium, allowing L-threonine, L-lysine, L-histidine, L-phenylalanine, L-arginine or L-glutamic acid to accumulate in the culture medium, and collecting L-threonine, L-lysine, L-histidine, L-phenylalanine, L-arginine or L-glutamic acid from the culture medium.

In the present invention, the cultivation, collection and purification of L-amino acids from the medium and the like may be performed by conventional fermentation methods wherein an L-amino acid is produced using a microorganism.

The culture medium may be either synthetic or natural, so long as the medium includes a carbon source and a nitrogen source and minerals, and if necessary, appropriate amounts of nutrients which the microorganism requires for growth. The carbon source may include various carbohydrates such as glucose, sucrose and xylose, and various organic acids. Depending on the mode of assimilation of the chosen microorganism, alcohols including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism may be used. As minerals, potassium monophosphate, magnesium sulfate, sodium chloride, ferrous sulfate, manganese sulfate, calcium chloride, and the like may be used. As vitamins, thiamine, yeast extract, and the like may be used. Additional nutrients may be added to the medium, if necessary. For example, if the microorganism requires an L-amino acid for growth (L-amino acid auxotrophy), a sufficient amount of the L-amino acid may be added to the cultivation medium.

The cultivation is performed preferably under aerobic conditions such as a shaking culture, and stirring culture with aeration, at a temperature of 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the target L-amino acid can be collected and purified by ion-exchange, concentration and/or crystallization methods.

### Examples

The present invention will be more concretely explained below with reference to the following non-limiting examples.

### Example 1: Substitution of the native promoter region of the xylE gene in E. coli by hybrid P_{L-tac} promoter

To substitute the native promoter region of the *xylE* gene, a DNA fragment carrying a hybrid P_{L-tac} promoter and chloramphenicol resistance marker (Cm^{R}) encoded by the *cat* gene was integrated into the chromosome of the *E. coli* MG1655 (ATCC 700926) in place of the native promoter region by the method described by Datsenko K.A. and Wanner B.L. (Proc.Natl.Acad.Sci.USA, 2000, 97, 6640-6645) which is also called as a "Red-mediated integration " and/or "Red-driven integration". The recombinant plasmid pKD46 (Datsenko, K.A., Wanner, B.L., Proc.Natl.Acad.Sci.USA, 2000, 97, 6640-6645) having a thermosensitive replicon was used as the donor of the phage λ-derived genes responsible for the Red-mediated recombination system. *Escherichia coli* strain BW25113 containing the recombinant plasmid pKD46 can be obtained from the *E. coli* Genetic Stock Center, Yale University, New Haven, USA, the accession number of which is CGSC7630.

The hybrid P_{L-tac} promoter was synthesized chemically. The nucleotide sequence of the substituted promoter is presented in the Sequence listing (SEQ ID NO: 3). The synthesized DNA fragment containing the hybrid P_{L-tac} promoter contains a *Bgl*II recognition site at the 5'-end thereof, which is necessary for further joining to the *cat* gene and 36 nucleotides homologous to the 5'-terminus of the *xylE* gene introduced for further integration into the bacterial chromosome.

A DNA fragment containing a Cm^{R} marker encoded by the *cat* gene was obtained by PCR using the commercially available plasmid pACYC184 (GenBank/EMBL accession number X06403, "Fermentas", Lithuania) as the template, and primers P1 (SEQ ID NO: 4) and P2 (SEQ ID NO: 5). Primer P1 contains a *Bgl*II recognition site at the 5'-end thereof, which is necessary for further joining to the hybrid P_{L-tac} promoter and primer P2 contains 36 nucleotides homologous to the region located 217 bp upstream of the start codon of the *xylE* gene, which was introduced into the primer for further integration into the bacterial chromosome.

PCR was provided using the "TermoHybaid PCR Express" amplificator. The reaction mixture (total volume - 50 µl) consisted of 5 µl of 10x PCR-buffer with 15 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added into the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C for 30 sec; and the final elongation for 7 min at +72 °C. Then, the amplified DNA fragment was purified by agarose gel-electrophoresis, extracted using "GenElute Spin Columns" ("Sigma", USA) and precipitated by ethanol.

Each of the two above-described DNA fragments was treated with *Bgl*II restrictase and ligated. The ligation product was amplified by PCR using primers P2 (SEQ ID NO: 5) and P3 (SEQ ID NO: 6). Primer P3 contains 36 nucleotides at 5'-end thereof which are homologous to the 5'-terminus of the *xylE* gene introduced for further integration into the bacterial chromosome.

The amplified DNA fragment was purified by agarose gel-electrophoresis, extracted using "GenElute Spin Columns" ("Sigma", USA) and precipitated by ethanol. The obtained DNA fragment was used for electroporation and Red-mediated integration into the bacterial chromosome of the *E. coli* MG1655/pKD46.

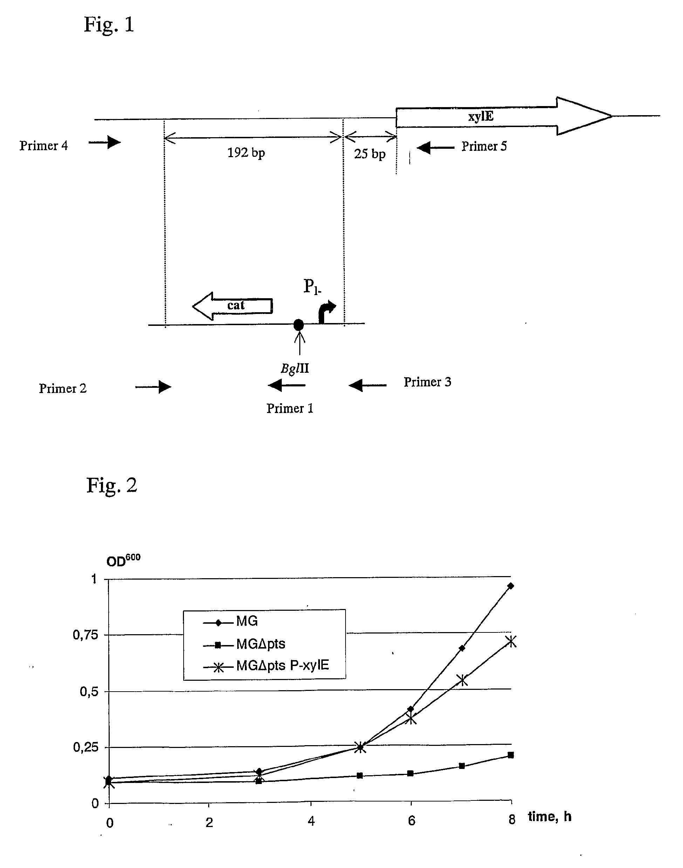

MG1655/pKD46 cells were grown overnight at 30 °C in the liquid LB-medium with the addition of ampicillin (100 µg/ml), then diluted 1:100 with the SOB-medium (Yeast extract, 5 g/l; NaCl, 0.5 g/l; Tryptone, 20 g/l; KCl, 2.5 mM; MgCl₂, 10 mM) with the addition of ampicillin (100 µg/ml) and L-arabinose (10 mM) (arabinose is used for inducing the plasmid encoding genes of the Red system) and grown at 30 °C to reach the optical density of the bacterial culture OD₆₀₀=0.4-0.7. Grown cells from 10 ml of the bacterial culture were washed 3 times with the ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated 2 hours at 37 °C, and then were spread onto L-agar containing 25 µg/ml of chloramphenicol. Colonies grown within 24 hours were tested for the presence of Cm^{R} marker, instead of the native promoter region of the *xylE* gene by PCR using primers P4 (SEQ ID NO: 7) and P5 (SEQ ID NO: 8). For this purpose, a freshly isolated colony was suspended in 20µl water and then 1µl of obtained suspension was used for PCR. The following temperature profile was used: initial DNA denaturation for 10 min at 95 °C; then 30 cycles of denaturation at 95 °C for 30 sec, annealing at 55 °C for 30 sec and elongation at 72 °C for 1 min; the final elongation for 7 min at 72 °C. A few Cm^{R} colonies tested contained the desired ∼2000 bp DNA fragment, confirming the presence of Cm^{R} marker DNA instead of 192 bp native promoter region of *xylE* gene (see Figure 1). One of these strains was cured from the thermosensitive plasmid pKD46 by culturing at 37 °C and the resulting strain was named as *E*. *coli* MG1655P_{L-tac}xylE.

### Example 2: Effect of increasing the xylE gene expression on growth of an E. coli strain having a disrupted PTS transport system

To show the effect of enhanced expression of the *xylE* gene on growth of an *E. coli* strain, the *E. coli* strain having a disrupted PTS transport system was constructed.

For that purpose, the DNA fragment carrying kanamycin resistance marker (Km^{R}) was integrated into the chromosome of the *E. coli* MG1655/pKD46 in place of the *ptsHI-crr* operon by the method described by Datsenko K.A. and Wanner B.L. (Proc.Natl.Acad.Sci.USA, 2000, 97, 6640-6645) which is also called as a "Red-mediated integration " and/or "Red-driven integration", also described in Example 1.

The *ptsHI-crr* operon has been elucidated (nucleotide numbers 2531786 to 2532043, 2532088 to 2533815 and 2533856 to 2534365 for *ptsH, ptsI* and *crr* genes, respectively, in the sequence of GenBank accession NC_000913.2, gi: 49175990). The *ptsHI-crr* operon is located between *cysK* and *pdxK* genes on the chromosome *of E. coli* K-12.

A DNA fragment carrying the Km^{R} gene was obtained by PCR using the commercially available plasmid pUC4KAN (GenBank/EMBL accession number X06404, "Fermentas", Lithuania) as the template and primers P6 (SEQ ID NO: 9) and P7 (SEQ ID NO: 10). Primer P6 contains 36 nucleotides homologous to the 5'-terminus of the *ptsH* gene and primer P7 contains 36 nucleotides homologous to the 3'-terminus of the *crr* gene. These sequences were introduced into primers P6 and P7 for further integration into the bacterial chromosome.

PCR was conducted as described in Example 1.

Then, the amplified DNA fragment was concentrated by agarose gel-electrophoresis, extracted from the gel by the centrifugation through "GenElute Spin Columns" ("Sigma", USA) and precipitated by ethanol. The obtained DNA fragment was used for electroporation and Red-mediated integration into the bacterial chromosome of the *E. coli* MG1655/pKD46 as described in Example 1, except that cells were spread after electroporation onto L-agar containing 50 µg/ml of kanamycin.

Colonies grown within 24 hours were tested for the presence of Km^{R} marker instead of *ptsHI*-*crr* operon by PCR using primers P8 (SEQ ID NO: 11) and P9 (SEQ ID NO: 12). For this purpose, a freshly isolated colony was suspended in 20µl water and then 1µl of the resulting suspension was used for PCR. PCR conditions were as described in Example 1. A few Km^{R} colonies tested contained the desired -1300 bp DNA fragment, which confirmed the presence of Km^{R} gene in the place of the *ptsHI*-*crr* operon. One of the obtained strains was cured from thermosensitive plasmid pKD46 by culturing at 37 °C and the resulting strain was named *E. coli* MG1655 *ΔptsHI-crr.*

Then, the DNA fragment from the chromosome of the above-mentioned *E. coli* MG1655P_{L-tac}xylE was transferred to *E. coli* MG1655 *ΔptsHI-crr* by P1 transduction (Miller, J.H. (1972) Experiments in Molecular Genetics, Cold Spring Harbor Lab. Press, Plainview, NY) giving the strain MG1655 Δ*ptsHI-crr* P_{L-tac}xylE.

The ability to grow on the minimal Adams with glucose (4%) as a carbon source was checked for the three *E*. *coli* strains MG1655, MG1655 *ΔptsHi-crr* and MG1655 *ΔptsHI-crr* P_{L-tac}xylE. As seen in Figure 2, *E. coli* MG1655 *ΔptsHI-crr* did not grow well (µ∼0.06) on the minimal Adams medium containing glucose. Increasing the *xylE* gene expression significantly enhanced the growing characteristics of recipient strains on the minimal Adams medium containing glucose.

### Example 3: Effect of increasing the xylE gene expression on threonine production

To test the effect of enhanced expression of the *xylE* gene which is under the control of P_{L-}tac promoter on threonine production, DNA fragments from the chromosome of the above-described *E. coli* MG1655P_{L-tac}xylE were transferred to the threonine-producing *E. coli* strain VKPM B-3996 by P1 transduction (Miller, J.H. (1972) Experiments in Molecular Genetics, Cold Spring Harbor Lab. Press, Plainview, NY). The strain VKPM B-3996 was deposited in Russian National Collection of Industrial Microorganisms (VKPM) (Russia, 117545 Moscow, 1^{st} Dorozhny proezd, 1) on April 7,1987 under the accession number B-3996.

Both *E. coli* strains B-3996 and B-3996P_{L-tac}xylE were grown for 18-24 hours at 37 °C on L-agar plates containing chloramphenicol (30 µg/ml). To obtain a seed culture, the strain was grown on a rotary shaker (250 rpm) at 32°C for 18 hours in 20x200 mm test tubes containing 2 ml of L-broth with 4% sucrose. Then, the fermentation medium was inoculated with 0.21 ml (10%) seed material. The fermentation was performed in 2 ml of minimal medium for fermentation in 20x200 mm test tubes. Cells were grown for 48 hours at 32°C with shaking at 250 rpm.

After cultivation, the amount of accumulated L-threonine in the medium was determined by paper chromatography using following mobile phase: butanol: acetic acid : water = 4 : 1 :1 (v/v). A solution (2%) of ninhydrin in acetone was used as a visualizing reagent. A spot containing L-threonine was cut off, L-threonine was eluted in 0.5 % water solution of CdCl₂, and the amount of L-threonine was estimated spectrophotometrically at 540 nm. The results are presented in Table 1. Threonine production was improved due to introduction of P_{L-tac}xylE.

The composition of the fermentation medium (g/l) is as follows:

| | |
|---|---|
| Glucose | 80.0 |
| (NH₄)₂SO₄ | 22.0 |
| NaCl | 0.8 |
| KH₂PO₄ | 2.0 |
| MgSO₄ 7H₂O | 0.8 |
| FeSO₄ 7H₂O | 0.02 |
| MnSO₄ 5H₂O | 0.02 |
| Thiamine HCl | 0.0002 |
| Yeast extract | 1.0 |
| CaCO₃ | 30.0 |

Glucose and magnesium sulfate are sterilized separately. CaCO₃ is dry-heat sterilized at 180°C for 2 hours. The pH is adjusted to 7.0. Antibiotic is introduced into the medium after sterilization.

**Table 1**

| Strain | OD₅₄₀ | Threonine, g/l |
|---|---|---|
| B3996P_{L-tac}xylE | 21.6 | 26.9 |
| | 19.5 | 24.0 |
| | 21.3 | 25.9 |
| | 23.4 | 26.4 |
| | 19.7 | 24.6 |
| | 22.0 | 29.4 |
| | 18.9 | 28.4 |
| | 20.0 | 25.9 |
| | 22.6 | 26.4 |
| | 20.8 | 27.1 |
| | 20.5 | 26.9 |
| | 19.5 | 25.9 |
| | 19.5 | 25.4 |
| | 20.2 | 23.6 |
| | 20.7 | 28.7 |
| | 20.2 | 28.9 |
| | 19.4 | 29.2 |
| | 20.5 | 29.0 |
| | 20.2 | 29.5 |
| | 20.5 | 29.2 |
| | 20.6 ± 1.1 | 27.1 ± 1.9 |
| B-3996 (control) | 16.5 | 21.0 |
| | 16.0 | 19.8 |
| | 16.0 | 19.0 |
| | 14.4 | 17.5 |
| | 15.5 | 19.0 |
| | 14.7 | 18.7 |
| | 16.6 | 20.6 |
| | 16.5 | 21.0 |
| | 14.7 | 16.0 |
| | 15.7 | 20.3 |
| | 15.7 ± 0.8 | 19.3 ± 1.6 |

### Example 4: Effect of increasing the xylE gene expression on L-lysine production

The whole nucleotide sequence of the chromosomal DNA *of E. coli* W3110 is already known (Science, 277, 1453-1474 (1997)). Based on the reported nucleotide sequence, primers were synthesized and the *xylE* gene was amplified by the PCR method as follows.

The chromosomal DNA was prepared by the conventional method (Sambrook, J., Fritsch E. F. and Maniatis T. (1989): Molecular Cloning: A laboratory manual, 2nd ed. Cold Spring Harbor Laboratory Press, Cold Spring Harbor, N.Y.). The primer 10 (SEQ ID NO: 13) was designed as a sequence in which an *Eco*RI recognition site is added to the 5'-terminal of the sequence 7479-7508 of the accession No. AE000476, and the primer 11 (SEQ ID NO: 14) was designed as a sequence complementary to the sequence in which a *Sal*I recognition site is added to the 5'-terminal of the sequence 8963-8992 of the accession No. AE000476. By using these primers, the *xylE* gene was amplified according to the standard conditions as described in "PCR protocols. Current methods and applications" (White, B.A., ed., Humana Press, Totowa, New Jersey,1993).

The PCR product was purified by a conventional method. The product was digested with restriction enzymes *Sal*I and *Eco*RI*,* and using a ligation kit, ligated to the vector pSTV29 which had been treated with the same restriction enzymes. Competent cells *of E. coli* JM109 were transformed with the ligation product (Sambrook, J., Fritsch E. F. and Maniatis T. (1989) Molecular Cloning: A laboratory manual, 2nd ed. Cold Spring Harbor Laboratory Press, Cold Spring Harbor, N.Y.), and the cells were plated on an L-plate (Bacto-trypton: 10 g/l yeast extract: 5 g/l NaCl: 5 g/l agar: 15 g/l pH 7.0) containing 10 *µ*g/ml of IPTG (isopropyl-ß-D-thiogalactopyranoside), 40 *µ*g/ml of X-Gal (5-bromo-4-chloro-3-indolyl-ß-D-galactoside) and 50*µ*g/ml of chloramphenicol and were cultured overnight. White colonies appeared and were picked up and isolated, and transformants were thus obtained. Plasmids were prepared from the transformants by the alkali-extraction method, and the plasmid pSTV29-xylE was obtained in which the *xylE* gene is linked to the *lac* promoter in the forward direction.

*E. coli* WC196 was used as an L-lysine producing strain belonging to the genus *Escherichia.*

WC196 was transformed with either the plasmid pSTV29-xylE or the vector pSTV29 and WC196/pSTV29-xylE and WC196/pSTV29 were obtained. Each of these strains was cultured in the L-medium containing 50 mg/l of chloramphenicol at 37 °C until the final OD at 600 nm reached around 0.6. Then an equal volume of 40 % glycerol solution was added to the culture, and the mixture was dispensed in an appropriate volume and stocked at -80 °C. This is hereafter called as a "glycerol stock".

In order to verify the effect of enhancing the xylose permease activity under L-lysine producing conditions, WC196 was transformed with the plasmids pSTV29-xylE and pCABD2 in accordance with the procedure as stated above. pCABD2 is a plasmid comprising a *dapA* gene coding for a dihydrodipicolinate synthase having a mutation which desensitizes feedback inhibition by L-lysine, a *lysC* gene coding for aspartokinase III having a mutation which desensitizes feedback inhibition by L-lysine, a *dapB* gene coding for a dihydrodipicolinate reductase, and a *ddh* gene coding for diaminopimelate dehydrogenase (US Patent 6,040,160). As a control, WC196 was transformed with the plasmids pSTV29 and pCABD2. Each of the obtained transformants was cultured in the L-medium containing 50 mg/l of chloramphenicol and 20 mg/l of streptomycin at 37 °C until the final OD at 600 nm reached around 0.6. Then an equal volume of 40 % glycerol solution was added to the culture, and the mixture was dispensed in an appropriate volume and stocked at -80 °C.

The glycerol stock of each of WC196/pSTV29-xylE and WC196/pSTV29 was melted and 100 *µ*l each was evenly plated on an L-plate containing 50 mg/l of chloramphenicol, and cultured at 37 °C for 24 hours. In addition, each of WC196/(pCABD2, pSTV29-xylE) and WC196/(pCABD2, pSTV29) was evenly plated on an L-plate containing 50 mg/l of chloramphenicol and 20 mg/l of streptomycin, and cultured at 37 °C for 24 hours. About one-eighth the amount of cells on the plate was inoculated into 20 ml of the fermentation medium containing the required drug(s) in a 500 ml-flask. The cultivation was carried out at 37 °C for 16 hours by using a reciprocal shaker at the agitation speed of 115 rpm. After the cultivation, the amounts of L-lysine and residual glucose in the medium were measured by a known method (Biotech-analyzer AS210, manufactured by Sakura Seiki Co.). And then the yield of L-lysine relative to the consumed glucose was calculated for each of the strains.

The composition of the fermentation medium (g/l) is as follows:

| | |
|---|---|
| Glucose | 40 |
| (NH₄)₂SO₄ | 24 |
| K₂HPO₄ | 1.0 |
| MgSO₄ x 7H₂O | 1.0 |
| FeSO₄ x 7H₂O | 0.01 |
| MnSO₄ x 5H₂O | 0.01 |
| Yeast extract | 2.0 |

pH is adjusted to 7.0 by KOH and the medium is autoclaved at 115°C for 10 min. Glucose and MgSO₄ x 7H₂O are sterilized separately. 30 g/l of CaCO₃, which has been dry-heat sterilized at 180°C for 2 hours, is added.

The results are shown in Table 2. WC196/pSTV29-xylE accumulated a higher amount of L-lysine as compared with WC196/pSTV29, in which the expression amount of xylose permease is not increased. In addition, it was observed that enhancing the xylose permease activity improves the accumulation and yield of L-lysine also in WC196/pCABD2, which produces L-lysine in a higher amount.

**Table 2**

| Strain | L-Lysine HCl, g/l | Yield from glucose (%) |
|---|---|---|
| WC196 /pSTV29 | 0.5 | 2.3 |
| WC196 /pSTV29-xylE | 1.0 | 3.5 |
| WC196 / pCABD2, pSTV29 | 1.8 | 32.4 |
| WC196 / pCABD2, pSTV29-xylE | 4.0 | 38.7 |

### Example 5: Effect of the increasing the xylE gene expression on L-arginine production

To test the effect of enhanced expression of the *xylE* gene which is under the control of P_{L-*tac*} promoter on arginine production, DNA fragments from the chromosome of the above-described *E. coli* MG1655P_{L-tac}xylE were transferred to the arginine-producing *E. coli* strain 382 by P1 transduction (Miller, J.H. (1972) Experiments in Molecular Genetics, Cold Spring Harbor Lab. Press, Plainview, NY). The strain 382 has been deposited in the Russian National Collection of Industrial Microorganisms (VKPM) (Russia, 117545 Moscow, 1^{st} Dorozhny proezd, 1) on April 10, 2000 under accession number VKPM B-7926.

The resulting strain 382 P_{L-tac}xylE and parent strain 382 were each cultivated at 32 °C for 18 hours in 2 ml of LB nutrient broth, and 0.3 ml of the obtained culture was inoculated into 2 ml of fermentation medium in a 20 x 200 mm test tube, and cultivated at 32 °C for 48 hours on a rotary shaker.

After the cultivation, the amount of L-arginine accumulated in the medium was determined by paper chromatography using following mobile phase: butanol: acetic acid : water = 4 : 1 : 1 (v/v). A solution (2%) of ninhydrin in acetone was used as a visualizing reagent. A spot containing L-arginine was cut off, L-arginine was eluted in 0.5 % water solution of CdCl₂, and the amount of L-arginine was estimated spectrophotometrically at 540 nm.

The composition of the fermentation medium (g/l) is as follows:

| | |
|---|---|
| Glucose | 48.0 |
| (NH₄)₂SO₄ | 35.0 |
| KH₂PO₄ | 2.0 |
| MgSO₄ x 7H₂O | 1.0 |
| Thiamine HCl | 0.0002 |
| Yeast extract | 1.0 |
| L-isoleucine | 0.1 |
| CaCO₃ | 5.0 |

Glucose and magnesium sulfate are sterilized separately. CaCO₃ is dry-heat sterilized at 180 °C for 2 hours. pH is adjusted to 7.0.

The results of 10 independent experiments are presented in Table 3. It can be seen from the Table 3, strain 382 P_{L-tac}xylE accumulated a higher amount of L-arginine as compared with strain 382, in which the expression amount of D-xylose permease is not increased.

**Table 3**

| Strain | OD₅₄₀ | Arginine, g/l |
|---|---|---|
| 382 | 17.0 | 4.9 |
| | 16.8 | 4.6 |
| | 16.8 | 4.9 |
| | 19.5 | 6.2 |
| | 21.1 | 5.3 |
| | 16.3 | 4.7 |
| | 16.1 | 4.6 |
| | 17.1 | 4.6 |
| | 17.6 | 4.9 |
| | 17.0 | 4.7 |
| | 17.5 ± 1.6 | 4.9 ± 0.5 |
| 382 P_{L-tac}xylE | 18.0 | 9.6 |
| | 17.8 | 11.9 |
| | 20.3 | 7.4 |
| | 19.2 | 8.6 |
| | 21.3 | 7.7 |
| | 20.1 | 7.0 |
| | 19.7 | 6.6 |
| | 20.6 | 7.6 |
| | 20.0 | 6.9 |
| | 20.6 | 8.4 |
| | 19.8 ± 1.1 | 8.2 ± 1.6 |

### Example 6: Production of L-histidine by L-histidine producing bacterium from fermentation of a mixture of glucose and xylose

The L-histidine-producing *E. coli* strain 80 was used for production of L-histidine by fermentation of a mixture of glucose and xylose. *E. coli* strain 80 (VKPM B-7270) is described in detail in Russian patent RU2119536. The strain 80 was deposited in the Russian National Collection of Industrial Microorganisms (VKPM; Dorozhny proezd. 1, Moscow 117545, Russian Federation) on October 15, 1999 under accession number B-7270. Then, It was then converted to an international deposit under the provisions of Budapest Treaty on June 12, 2004.

To test the effect on histidine production of enhanced expression of the *xylE* gene which is under the control of P_{L-*tac*} promoter, the DNA fragments from the chromosome of the above-described *E. coli* MG1655P_{L-tac}xylE were transferred to histidine-producing *E. coli* strain 80 by P1 transduction (Miller, J.H. (1972) Experiments in Molecular Genetics, Cold Spring Harbor Lab. Press, Plainview, NY). Transformation of strain 80 and the resulting strain 80 P_{L-tac}xylE with the pMW119mod-xylA-R plasmid was performed by ordinary methods, yielding strains 80/pMW119mod-xylA-R and 80 P_{L-tac}xylE/pMW119mod-xylA-R. Cloning of the *xylABFGHR* locus from the chromosome *of E. coli* strain MG1655 is described in the Russian patent application RU2005106720.

To obtain the seed culture, both strains 80/pMW119mod-xylA-R and 80 P_{L-tac}xylE/pMW119mod-xylA-R, were grown on a rotary shaker (250 rpm) at 27 °C for 6 hours in 40 ml test tubes (Ø 18 mm) containing 2 ml of L-broth with 1 g/l of streptomycin and 100 mg/l ampicillin. Then, 2 ml (5%) of seed material was inoculated into the fermentation medium. Fermentation was carried out on a rotary shaker (250 rpm) at 27 °C for 50 hours in 40 ml test tubes containing 2 ml of fermentation medium.

After cultivation, the amount of L-histidine which had accumulated in the culture medium was determined by paper chromatography. The composition of the mobile phase is the following: butanol: acetate: water = 4 : 1: 1 (v/v). A solution (0.5%) of ninhydrin in acetone was used as a visualizing reagent. The results are presented in Table 4.

The composition of the fermentation medium (g/l) is as follows:

| | |
|---|---|
| Carbohydrates (total) | 100.0 |
| Mameno | 0.2 of total nitrogen |
| (Soybean hydrolysate) | |
| L-proline | 0.8 |
| (NH₄)₂SO₄ | 25.0 |
| K₂HPO₄ | 2.0 |
| MgSO₄x 7H₂O | 1.0 |
| FeSO₄ x 7H₂O | 0.01 |
| MnSO₄ x 5H₂O | 0.01 |
| Thiamine HCl | 0.001 |
| Betaine | 2.0 |
| CaCO₃ | 6.0 |
| Streptomycin | 1.0 |

Carbohydrates (glucose, xylose), L-proline, betaine and magnesium sulfate are sterilized separately. CaCO₃ is dry-heat sterilized at 110 °C for 30 min. pH is adjusted to 6.0 by KOH before sterilization.

**Table 4**

| Strain | CT, hours | Growth, A₄₅₀ | Residual glucose, % | Residual xylose, % | Amount of histidine, g/l |
|---|---|---|---|---|---|
| 80/pMW119mod-xylA-R | 0 | - | 100 | 100 | - |
| | 16 | 15 | 85.6 | 92.3 | 0.3+0.01 |
| | 24 | 48 | 44.3 | 79.8 | 1.1+0.08 |
| | 40 | 67 | 0.86 | 14.7 | 5.9+0.05 |
| | 48 | 66 | <0.5 | 0.7 | 6.5+0.3 |
| 80 P_{1-tac}xylE/ pMW119mod-xylA-R | 0 | - | 100 | 100 | - |
| | 16 | 15 | 90.9 | 89.3 | 0.3+0.01 |
| | 24 | 25 | 78.6 | 72.9 | 0.7+0.03 |
| | 40 | 69 | 26.3 | <0.5 | 6.3+0.3 |
| | 48 | 66 | 2.5 | <0.5 | 7.4+0.3 |

It can be seen from Table 4 that strain 80 P_{L-tac}xylE/pMW119mod-xylA-R caused accumulation of a higher amount of L-histidine in the medium containing glucose and xylose mixture as compared with strain 80/pMW119mod-xylA-R, in which the expression of D-xylose permease is not increased.

While the invention has been described in detail with reference to preferred embodiments thereof, it will be apparent to one skilled in the art that various changes can be made within the scope of the claims.

### Industrial Applicability

Present invention provides a method for producing non-aromatic or aromatic L-amino acids such as L-threonine, L-lysine, L-histidine, L-phenylalanine, L-arginine and L-glutamic acid by fermentation. According to the present invention, productivity of L-amino acid-producing bacteria of the *Enterobacteriaceae* family can be enhanced.

## Claims

1. A method for producing an L-amino acid which comprises cultivating an L-amino acid-producing bacterium of the *Enterobacteriaceae* family in a culture medium containing glucose to cause accumulation of the L-amino acid in the culture medium, and isolating the L-amino acid from the culture medium, wherein said bacterium has been modified to increase the expression of xylE gene encoding D-xylose permease, and wherein the expression of xylE gene is increased by modifying an expression control sequence of the xylE gene or by increasing the copy number of the xylE gene.

2. The method according to claim 1, wherein the expression of xylE gene is increased by placing the xylE gene under the control of a potent promoter selected from the group consisting of *lac* promoter, trp promoter, trc promoter, and P_{R} or P_{L} promoter of lambda phage.

3. The method according to claim 1 or 2, wherein said bacterium has been additionally modified to enhance an activity of glucokinase.

4. The method according to any one of claims 1 to 3, wherein said bacterium has been additionally modified to enhance an activity of xylose isomerase.

5. The method according to any one of claims 1 to 4, wherein said bacterium has been additionally modified to increase the expression of the *xylABFGHR* locus.

6. The method according to any one of claims 1 to 5, wherein said bacterium is selected from the group consisting of the genera *Escherichia, Enterobacter, Erwinia, Klebsiella, Pantoea, Providencia, Salmonella, Serratia, Shigella,* and *Morganella.*

7. The method according to any one of claims 1 to 5, wherein said bacterium is *Escherichia coli.*

8. The method according to any one of claims 1 to 7, wherein said xylE gene encodes a D-xylose permease selected from the group consisting of:
(A) a protein which comprises the amino acid sequence of SEQ ID NO: 2; and
(B) a variant protein of the amino acid sequence shown in SEQ ID NO: 2, which has a homology of not less than 80% with respect to the entire amino acid sequence of SEQ ID NO: 2, and which has an activity of D-xylose permease and results in enhanced production of an L-amino acid.

9. The method according to any one of claims 1 to 7, wherein said xylE gene comprises a DNA selected from the group consisting of:
(A) a DNA which comprises a nucleotide sequence of nucleotides 1 to 1476 in SEQ ID NO: 1; and
(B) a DNA which is hybridizable with a nucleotide sequence of nucleotides 1-1476 in SEQ ID NO: 1, or a probe which can be prepared from said nucleotide sequence under stringent conditions, and encodes a protein having an activity of D-xylose permease, wherein said stringent conditions comprise those in which washing is performed at 60° C at a salt concentration of 1 xSSC and 0.1 % SDS for 15 minutes.

10. The method according to any one of claims 1 to 9, wherein said L-amino acid is selected from a group consisting of L-threonine, L-lysine, L-histidine, L-phenylalanine, L-arginine and L-glutamic acid.

11. A method for producing L-threonine which comprises cultivating L-threonine producing *Escherichia coli* in a culture medium containing glucose to cause accumulation of the L-threonine in the culture medium, and isolating the L-threonine from the culture medium, wherein said *Escherichia coli* has been modified to increase the expression of xylE gene coding for the amino acid sequence of SEQ ID NO: 2, and wherein said expression of xylE gene is increased by placing the xylE gene under the control of a potent promoter.

12. The method according to claim 11, wherein said xylE gene comprises the nucleotide sequence of SEQ ID NO: 1.

## Patentansprüche

1. Verfahren zur Produktion einer L-Aminosäure, welches Kultivieren eines L-Aminosäure-produzierenden Bakteriums der Enterobacteriaceae-Familie in einem Kulturmedium, enthaltend Glucose, um die Anreicherung der L-Aminosäure in dem Kulturmedium auszulösen, und Isolieren der L-Aminosäure aus dem Kulturmedium umfasst, wobei das genannte Bakterium modifiziert wurde, um die Expression des die D-Xylose-Permease kodierenden xylE-Gens zu steigern, und wobei die Expression des xylE-Gens durch Modifizieren einer Expressions-Kontrollsequenz des xylE-Gens oder durch Steigern der Kopienzahl des xylE-Gens gesteigert wird.

2. Verfahren gemäß Anspruch 1, wobei die Expression des xylE-Gens gesteigert wird, indem das xylE-Gen der Kontrolle eines potenten Promotors, ausgewählt aus der Gruppe, bestehend aus lac-Promotor, trp-Promotor, trc-Promotor und P_{R}- oder P_{L}-Promotor der lambda-Phage, unterstellt wird.

3. Verfahren gemäß Anspruch 1 oder 2, wobei das genannte Bakterium zusätzlich modifiziert wurde, um eine Aktivität der Glycokinase zu verbessern.

4. Verfahren gemäß einem der Ansprüche 1 bis 3, wobei das genannte Bakterium zusätzlich modifiziert wurde, um eine Aktivität der Xylose-Isomerase zu verbessern.

5. Verfahren gemäß einem der Ansprüche 1 bis 4, wobei das genannte Bakterium zusätzlich modifiziert wurde, um die Expression des xylABFGHR-Locus zu steigern.

6. Verfahren gemäß einem der Ansprüche 1 bis 5, wobei das genannte Bakterium aus der Gruppe, bestehend aus den Gattungen Escherichia, Enterobacter, Erwinia, Klebsiella, Pantoea, Providencia, Salmonella, Serratia, Shigella und Morganella, ausgewählt ist.

7. Verfahren gemäß einem der Ansprüche 1 bis 5, wobei das genannte Bakterium Escherichia coli ist.

8. Verfahren gemäß einem der Ansprüche 1 bis 7, wobei das genannte xylE-Gen eine D-Xylose-Permease kodiert, ausgewählt aus der Gruppe bestehend aus:
(A) ein Protein, das die Aminosäuresequenz SEQ ID NO: 2 umfasst; und
(B) eine Proteinvariante der Aminosäuresequenz, die in SEQ ID NO: 2 dargestellt ist, die eine Homologie von nicht weniger als 80 % in Bezug auf die gesamte Aminosäuresequenz SEQ ID NO: 2 aufweist und die eine D-Xylose-Permease-Aktivität aufweist und auf die eine verbesserte L-Aminosäureproduktion zurückzuführen ist.

9. Verfahren gemäß einem der Ansprüche 1 bis 7, wobei das genannte xylE-Gen eine DNA umfasst, ausgewählt aus der Gruppe, bestehend aus:
(A) eine DNA, die eine Nukleotidsequenz der Nukleotide 1 bis 1476 in SEQ ID NO: 1 umfasst; und
(B) eine DNA, die mit einer Nukleotidsequenz der Nukleotide 1 bis 1476 in SEQ ID NO: 1 hybridisierbar ist, oder eine Sonde, die aus der genannten Nukleotidsequenz unter stringenten Bedingungen hergestellt werden kann, und ein Protein mit einer D-Xylose-Permease-Aktivität kodiert, wobei die genannten stringenten Bedingungen diejenigen umfassen, bei denen Waschen bei 60°C mit einer Salzkonzentration von 1 xSSC und 0,1 % SDS für 15 Minuten durchgeführt wird.

10. Verfahren gemäß einem der Ansprüche 1 bis 9, wobei die genannte L-Aminosäure aus der Gruppe, bestehend aus L-Threonin, L-Lysin, L-Histidin, L-Phenylalanin, L-Arginin und L-Glutaminsäure, ausgewählt ist.

11. Verfahren zur Produktion von L-Threonin, welches Kultivieren von L-Threonin-produzierenden Escherichia coli in einem Kulturmedium, enthaltend Glucose, um die Anreicherung des L-Threonins in dem Kulturmedium auszulösen, und Isolieren des L-Threonins aus dem Kulturmedium umfasst, wobei die genannten Escherichia coli modifiziert wurden, um die Expression des die Aminosäuresequenz SEQ ID NO: 2 kodierenden xylE-Gens zu steigern, und wobei die genannte Expression des xylE-Gens gesteigert wird, indem das xylE-Gen der Kontrolle eines potenten Promotors unterstellt wird.

12. Verfahren gemäß Anspruch 11, wobei das genannte xylE-Gen die Nukleotidsequenz SEQ ID NO: 1 umfasst.

## Revendications

1. Procédé de production d'un acide L-aminé qui comprend le fait de mettre en culture une bactérie produisant un acide L-aminé de la famille des *Enterobacteriaceae* dans un milieu de culture contenant du glucose pour provoquer l'accumulation de l'acide L-aminé dans le milieu de culture, et d'isoler l'acide L-aminé à partir du milieu de culture, où ladite bactérie a été modifiée pour augmenter l'expression du gène xylE codant pour la D-xylose perméase, et où l'expression du gène xylE est augmentée en modifiant une séquence de contrôle d'expression du gène xylE ou en augmentant le nombre de copies du gène xylE.

2. Procédé selon la revendication 1, dans lequel l'expression du gène xylE augmente en plaçant le gène xylE sous le contrôle d'un promoteur puissant choisi dans le groupe constitué du promoteur lac, du promoteur trp, du promoteur trc, et du promoteur P_{R} ou P_{L} du phage lambda.

3. Procédé selon la revendication 1 ou 2, dans lequel ladite bactérie a été en outre modifiée pour améliorer une activité de glucokinase.

4. Procédé selon l'une quelconque des revendications 1 à 3, dans lequel ladite bactérie a été en outre modifiée pour améliorer une activité de xylose isomérase.

5. Procédé selon l'une quelconque des revendications 1 à 4, dans lequel ladite bactérie a été en outre modifiée pour augmenter l'expression du locus *xylABFGHR.*

6. Procédé selon l'une quelconque des revendications 1 à 5, dans lequel ladite bactérie est choisie dans le groupe constitué des genres *Escherichia, Enterobacter, Erwinia, Klebsiella, Pantoea, Providencia, Salmonella, Serratia, Shigella,* et *Morganella.*

7. Procédé selon l'une quelconque des revendications 1 à 5, dans lequel ladite bactérie est une *Escherichia coli.*

8. Procédé selon l'une quelconque des revendications 1 à 7, dans lequel ledit gène xylE code pour une D-xylose perméase choisie dans le groupe constitué :
(A) d'une protéine qui comprend la séquence d'acides aminés de la séquence SEQ ID NO : 2 ; et
(B) d'une protéine variante de la séquence d'acides aminés présentée dans la séquence SEQ ID NO : 2, qui a une homologie de pas moins de 80% par rapport à la séquence d'acides aminés entière de la séquence SEQ ID NO : 2, et qui a une activité de D-xylose perméase et conduit à une production améliorée d'un acide L-aminé.

9. Procédé selon l'une quelconque des revendications 1 à 7, dans lequel ledit gène xylE comprend un ADN choisi dans le groupe constitué :
(A) d'un ADN qui comprend une séquence nucléotidique des nucléotides 1 à 1476 dans la séquence SEQ ID NO : 1 ; et
(B) d'un ADN qui peut s'hybrider avec une séquence nucléotidique des nucléotides 1 à 1476 dans la séquence SEQ ID NO : 1, ou une sonde qui peut être préparée à partir de ladite séquence nucléotidique dans des conditions rigoureuses, et qui code pour une protéine ayant une activité de D-xylose perméase, où lesdites conditions rigoureuses comprennent celles dans lesquelles le lavage est réalisé à 60°C à une concentration de sel de 1 xSSC et de SDS à 0,1% pendant 15 minutes.

10. Procédé selon l'une quelconque des revendications 1 à 9, dans lequel ledit acide L-aminé est choisi dans un groupe constitué de L-thréonine, de L-lysine, de L-histidine, de L-phénylalanine, de L-arginine et de L-acide glutamique.

11. Procédé de production de L-thréonine qui comprend le fait de mettre en culture de *l'Escherichia coli* produisant la L-thréonine dans un milieu de culture contenant du glucose pour provoquer l'accumulation de la L-thréonine dans le milieu de culture, et d'isoler la L-thréonine à partir du milieu de culture, où ladite *Escherichia coli* a été modifiée pour augmenter l'expression du gène xylE codant pour la séquence d'acides aminés de la séquence SEQ ID NO : 2, et où ladite expression du gène xylE est augmentée en plaçant le gène xylE sous le contrôle d'un promoteur puissant.

12. Procédé selon la revendication 11, dans lequel ledit gène xylE comprend la séquence nucléotidique de la séquence SEQ ID NO : 1.
